# EUROPEAN PATENT SPECIFICATION

(11) **EP 2 519 140 B1**
(45) Date of publication and mention of the grant of the patent: **24.01.2018**
(21) Application number: 10795895.1
(22) Date of filing: 15.12.2010
(51) Int. Cl.: A61B 1/005, A61B 1/06, A61B 1/05, A61B 1/303

(54) **DISPOSABLE PROBE FOR HYDROTHERMAL ABLATION**
EINWEG-SONDE FÜR HYDROTHERMISCHE ABLATION
SONDE JETABLE POUR ABLATION HYDROTHERMIQUE

(30) Priority: 31.12.2009 US 291507 P
(43) Date of publication of application: 07.11.2012
(73) Proprietor: Boston Scientific Scimed, Inc., Natick, MA 01760-1537 (US)
(72) Inventor: SEEHUSEN, Ashley, Newton, Massachusetts 02458 (US); OSTROVSKY, Isaac, Wellesley, Massachusetts 02481 (US)
(74) Representative: Pfenning, Meinig & Partner mbB
(86) International application number: PCT/US2010/060455
(87) International publication number: WO 2011/081963

(56) References cited:
- US-A- 4 836 189
- US-A- 5 179 934
- US-A1- 2004 267 095
- US-A1- 2007 055 103
- US-A1- 2008 015 618
- US-A1- 2009 198 106
- US-B1- 6 277 064

## Description

### Priority Claim

This application claims the priority to the U.S. Provisional Application Serial No. 61/291,507, entitled " *Disposable Probe for Hydrothermal Ablation with Digital Miniature Camera and Articulating Distal Section"* filed on December 31, 2009.

### Background

Devices for the treatment of menorrhagia comprise ablation of the uterine lining using heated fluid supplied to the uterus by a supply lumen and withdrawn therefrom via a return lumen. Such treatments rely heavily on the maintenance of a substantially continuous fluid flow within the uterus to ensure the safety, efficiency and effectiveness of the treatment. Visualization of the target ablation site is vital to ensure that this continuous circulating flow is maintained as well as to monitor the ablation of various locations. Presently available systems employ rigid or semi-rigid hysteroscopes which provide visualization via a series of optical fibers (e.g. located within an endoscope or embedded within a hysteroscope) advanced to a target location in the body. Such scopes generally include a Charge Couple Camera ("CCD") at a proximal end thereof which remains outside the body. Light from the distal end of the scope is transmitted to the CCD via a group of optical fibers which extend through the scope to the distal end thereof. The optical fibers require significant space, which increase the size of the insertion section of the scope. In addition, the bulk of the camera makes manipulation of the scope more difficult and fatiguing for the user. US 4836189 describes an endoscopic system according to the preamble of claim 1. US 2007/055103 A1 shows an endoscope comprising a distal head which includes an imaging system. The imaging system is connected to a first and second shaft portion in an articulating manner to be movable. The endoscope comprises an elongated shaft configured to be inserted into a lumen and which includes a shaft lumen.
US 6 277 064 B1 discloses a surgical instrument having an offset endoscope with an articulated ball-joint mechanism. The surgical instrument includes an elongated shaft including several lumens, wherein the endoscope extends through one of the lumens from its proximal to its distal end. The endoscope lumen is not open to lumen of the shaft.
US 2004/0267095 and US 2009/0198106 disclose an endoscope removably attached to a distal end of a main body. The main body has an elongated form.

### Summary of the Invention

The present invention is directed to a device, comprising an elongated shaft configured for insertion through a body lumen to a desired position within a target body organ, the shaft defining a first shaft lumen extending therethrough and an articulating section located at a distal end of the shaft, the articulating section being movable out of longitudinal alignment with the shaft under user control, the articulating section comprising a first articulating section lumen extending therethrough from a proximal end open to the first shaft lumen to a distal opening in a distal face of the articulating section in combination with a camera mounted on a distal face of the articulating section and a data transmission wire extending through the shaft between a distal end connected to the camera and a proximal end which remains external to the body for attachment to an external image processing device. A light source at the distal face of the articulating section is aimed to illuminate an area within a field of view of the camera.

### Brief Description of the Drawings

The accompanying drawings, which are incorporated in and constitute a part of this specification, illustrate several embodiments of the invention and, together with the description, serve to explain the principles of the invention. In the drawings:

Fig. 1 shows a perspective partial zoom view of a system according to a first exemplary embodiment of the present invention; and
Fig. 2 shows a perspective view of the system of Fig. 1 in a first operative configuration wherein an instrument channel thereof is deflated.

### Detailed Description

The present invention may be further understood with reference to the following description and the appended drawings. The present invention relates to a system and method facilitating endolumenal procedures within living bodies. More specifically, the present invention relates to a flexible instrument insertable into an internal organ such as the uterus to permit visualization of target locations within the organ while facilitating the insertion of fluids and/or devices for operation within the organ. An instrument according to the present invention includes a distal articulating and telescoping section which may optionally be removable therefrom facilitating visualization and access to a wide range of areas across a plurality of planes. The distal articulating section permits observation of various target areas without moving the entire device as was required with previous rigid and semi-rigid devices, as will be described in greater detail hereinafter. The device according to the invention further comprises a miniature digital camera at a distal end thereof connected to the external portions of the device via electrical leads - not optical fibers - thus leaving more space for operative components of the device and/or for a working channel extending therethrough. Although the exemplary system and method of the present invention are described with respect to devices for ablating the endometrium, those skilled in the art will understand that the present invention, and/or components thereof, may be utilized in conjunction with devices for the treatment of any internal organ. For example, such devices may be employed in prostate treatment (microwave or cryoablation), irrigation systems, with other devices for procedures which infuse heated fluids to the body or for any device requiring the insertion of an endoscopic instrument into a living body.

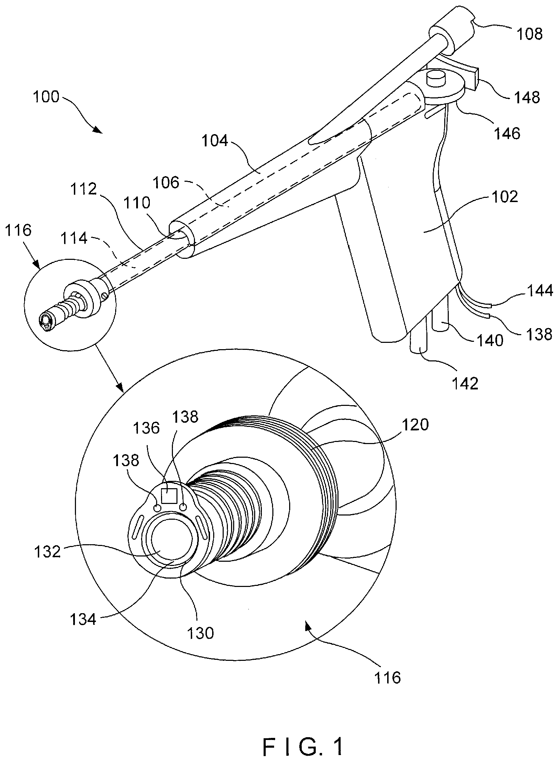

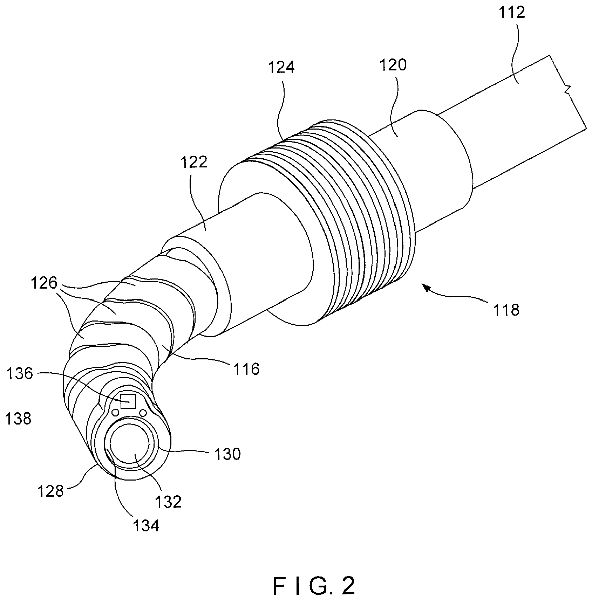

Figs. 1 - 2 depict an exemplary device 100 according to the present invention. The device 100 includes an insertion section configured to be advanced to a target position within a living body (e.g., via a body lumen accessed via a naturally occurring body orifice). In this embodiment, the device 100 is an ablation probe for hydrothermal ablation ("HTA") comprising a handle 102 and an elongated cannula 104 extending distally therefrom. The cannula 104 includes a lumen 106 extending therethrough from a proximal opening 108 to a distal opening 110 configured for engagement with a reduced diameter channel 112 extending distally therefrom. Specifically, the channel 112 may be attached to a distal end of the cannula 104 or, in another embodiment, may extend through the cannula 104. A distal end of the channel 112 is configured to receive an articulating section 116 having a lumen 134 extending therethrough in alignment with and open to the lumen 114. The distal end of the channel 112 includes a seal 118 including a plurality of flexible fins extending radially outward from the channel 112 to a diameter selected so that, when the articulating section 116 is located in a desired position within the uterus, the fins of the seal 118 engage the wall of the cervix and/or the cervical os to seal the uterus so that heated fluids supplied thereto via the articulating section do not leak into the cervix. Those skilled in the art will understand that the flexibility of the seal 118 allows the fins to flex proximally as the device 100 is inserted distally through the cervix facilitating passage of the device 100 therethrough. When the articulating section 116 enters the uterus, the bias of the material returns the fins of the seal 118 to their original position (e.g., with each fin extending in a plane substantially perpendicular to a longitudinal axis of the articulating section 116). The user then draws the device 100 proximally until the seal 118 is seated in the cervical os with the fins of the seal 118 pressing against the tissue of the os under the bias of the material of which they are formed to seal the cervix and prevent fluids from leaking from the uterus into the cervix. The seal 118 may be formed of silicone and the articulating section 116 may be formed of one of a plastic (e.g., polypropylene, polyethylene, nylon, etc.) and a metal (e.g., stainless steel). Engagement of the seal 118 with the channel 112 and the articulating section 116 may be facilitated by one of a threaded engagement, a friction fit, bonding or another temporary attachment means known in the art.

The articulating section 116 is formed as a series of substantially cylindrical links 126 attached to one another to define a channel 130 extending therethrough. Specifically, each of the links 126 tapers down in diameter toward a distal end thereof, with the decrease in diameter permitting insertion of the distal end of each link 126 into a proximal opening of an adjacent one of the links 126. A proximal end of each of the links in this embodiment includes a circumferential tab (not shown) configured to engage a corresponding circumferential groove (not shown) formed in a distal end of an adjacent link 126 wherein a diameter of the circumferential groove (not shown) is larger than a diameter of the proximal end of the adjacent link to permit the movement of adjacent links relative to one another. Specifically, in an exemplary embodiment, adjacent links may be movable out of longitudinal alignment with one another to permit the articulating section 116 to exhibit an overall curvature conforming to, for example, a body conduit into which the articulating section 116 is inserted. In an exemplary embodiment, each link 126 is configured to be deflectable by up to 15° from an adjacent link 126. In the embodiment of Figs. 1 - 2, the articulating section 116 comprises six links 126 and may therefore deflect by up to ±90° from a longitudinal axis of the channel 112. The desired deflection angle may therefore be changed by adding or reducing the number of links 126 in the articulating section 116. This engagement permits each of the links 126 to be at least partially receivable within an adjacent link 126. Thus, the articulating section 116 may have at least two degrees of freedom, permitting a curvature thereof in any direction along a single plane or, in another embodiment, in any direction with respect to a longitudinal axis of the articulating section 116. The articulating section 116 may be formed substantially similarly to the device described in U.S. Application Serial No. 12/121,345 entitled "Articulating Torqueable Hollow Device" filed on May 15, 2008 to Ostrovsky et al., the contents of which are incorporated herein by reference.

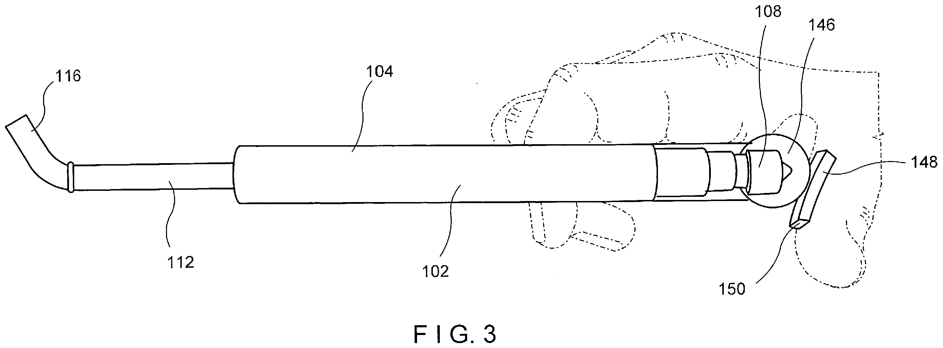

Movement of the articulating section 116 is facilitated by a control wheel 146 located on a proximal portion of the handle 102, the control wheel being connected to a knob 148 which may be actuated by a user of the device 100. Specifically, the control wheel 146 is connected to two pull wires (not shown) extending through specially formed channels extending through the handle, lumen 114 and each link of the articulating section 116. As shown in Fig. 3, manual actuation of a lateral side of the knob 148 rotates the control wheel 146 which in turn places a pressure on the control wires (not shown) causing lateral movement of the articulating section 116. Specifically, Fig. 3 depicts actuation of a left lateral wall 150 of the knob 148 causing the control wheel to turn clockwise shortening a length of the control wire on the right lateral portion of the device 100. This shortening causes the articulating section 116 to extend outward toward the right lateral portion of the device 100.

Each of the channel 112 and the articulating section 116 defines two lumens extending therethrough - e.g., one for the introduction of fluids into the target organ and one for the withdrawal of fluids therefrom. Specifically, as shown in Fig. 1, the device 100 comprises an introduction lumen 132 located within and surrounded by a withdrawal lumen 134. In this embodiment, the introduction lumen 132 is concentric with the withdrawal lumen 134. A proximal end of the introduction lumen 132 is connected to a fluid input port 140 of the handle 102 and a proximal end of the withdrawal lumen 134 is connected to the lumen 114 of the channel 112 which extends to a fluid withdrawal port 142 at a proximal end thereof.

A distal end of the articulating section 116 according to this embodiment has an increased outer diameter end link 128 while a diameter of the withdrawal lumen 134 extending through the articulating section 116 remains substantially constant. A miniature camera 136 is mounted on a distal face of an outer wall of the end link 128. In a preferred embodiment, the digital camera is approximately 1mm × 1mm in size although other sizes may be employed without deviating from the scope of the present invention. The digital camera 136 is preferably a Complimentary Metal-Oxide Semiconductor ("CMOS") camera. Fiber optic light conductors 138 also extend through the device 100 from the handle 102 where they are connected to a source of light (not shown) to the distal face of the end link 128 to provide illumination to an area visualized by the camera 136. The CMOS digital camera 136 according to the present invention transmits recorded data to a processor such as a computer via a signal cable 144, wherein the processor may be a computer or other electronic device. It is noted that although the present invention has been described with a fiber optic light source, any other light source may be employed without deviating from the spirit and scope of the present invention including, but not limited to light emitting diodes (LEDs).

The exemplary articulating section 116 of the present invention bypasses the need for optical conductors and a conventional CCD camera, thus allowing a reduction of a diameter of the distal end of the device 100 as compared to conventional devices employing imaging systems using a bundle of optical fibers to collect an image at the distal end and to transmit this image to a camera which remains external to the body. By employing a CMOS camera 136, the device of the present invention permits a miniaturization of the device not possible with presently available endoscopic devices. Furthermore, since the CMOS camera 136 is less expensive than conventional endoscopic cameras, the exemplary articulating section 116 of the present invention may be configured to be disposable and therefore eliminates a need for sterilization, repair, etc. As a result, the device 100 of the present invention is both smaller and lighter than conventional imaging devices. Specifically, the articulating section 116 according to the present invention permits the use of a smaller outer diameter of the end link 128 while still providing at least as large as inner diameter as is used with presently available device. In an alternate diameter, the outer diameter of the articulating section 116 may be the same as conventional devices but may provide a larger inner diameter of the lumen 134 extending therethrough to permit the insertion of larger tools therethrough or to increase a volume of fluid flow therethrough. In a preferred embodiment, an outer diameter of the articulating section 116 is approximately 6 mm. whereas conventional devices have an outer diameter of approximately 8 mm. The articulating section 116 of the present invention has the added advantage of being disposable and inexpensive in comparison to conventional imaging devices which also must be sterilized for reuse.

Those skilled in the art will understand that the described exemplary embodiments of the present invention may be altered without departing from the spirit or scope of the invention. Thus, it is to be understood that these embodiments have been described in an exemplary manner and are not intended to limit the scope of the invention which is intended to cover all modifications and variations of this invention that come within the scope of the appended claims and their equivalents.

The present subject-matter includes, inter alia, the following aspects:
1. A device, comprising:
   an elongated shaft configured for insertion through a body lumen to a desired position within a target body organ, the shaft defining a first shaft lumen extending therethrough;
   an articulating section located at a distal end of the shaft, the articulating section being movable out of longitudinal alignment with the shaft under user control, the articulating section comprising a first articulating section lumen extending therethrough from a proximal end open to the first shaft lumen to a distal opening in a distal face of the articulating section;
   a camera mounted on a distal face of the articulating section;
   a data transmission wire extending through the shaft between a distal end connected to the camera and a proximal end which remains external to the body for attachment to an external image processing device; and
   a light source at the distal face of the articulating section aimed to illuminate an area within a field of view of the camera.
2. The device of aspect 1, wherein the articulating section is movable in any direction along at least one plane.
3. The device of aspect 1, wherein movement of the articulating section is controlled by an actuator on a handle mounted to a proximal end of the shaft.
4. The device of aspect 3, wherein the actuator is formed as a control wheel operably connected to a control wire extending from the control wheel to a distal end of the articulating section.
5. The device of aspect 2, wherein the articulating section is formed of a plurality of substantially cylindrical elements linked together so as to provide a substantially cylindrical articulating section.
6. The device of aspect 5, wherein each of the cylindrical elements is movable out of longitudinal alignment with an adjacent one of the cylindrical elements.
7. The device of aspect 5, wherein the articulating section is deflectable by approximately 90° from a longitudinal axis of the elongated shaft.
8. The device of aspect 1, further comprising a seal configured to prevent leakage of fluids from a target body cavity, the seal being configured to permit temporary attachment of the articulating section to the shaft.
9. The device of aspect 5, wherein the seal includes a plurality of flexible disks extending radially outward from an outer surface of the shaft.
10. The device of aspect 1, further comprising a second shaft lumen extending through the shaft and wherein the articulating section further comprises a second articulating section lumen extending between a proximal end open to a distal end of the second shaft lumen and a distal opening at a distal end of the articulating section.
11. The device of aspect 7, wherein the shaft is sized and shaped for insertion through a cervix into a uterus for hydrothermal ablation.
12. The device of aspect 1, wherein the camera is a Complimentary Metal-Oxide Semiconductor ("CMOS").
13. The device of aspect 9, wherein the camera is no greater than 1 mm. x 1 mm. in size.
14. The device of aspect 11, wherein an outer diameter of the articulating section is no greater than 6 mm.

## Claims

1. A device (100), comprising:
an elongated shaft configured for insertion through a body lumen to a desired position within a target body organ, the shaft defining a first shaft lumen (106) extending therethrough;
an articulating section (116) located at a distal end of the shaft, the articulating section
being movable out of longitudinal alignment with the shaft under user control, the
articulating section comprising a first articulating section lumen (132) extending therethrough
from a proximal end open to the first shaft lumen to a distal opening in a distal face of the articulating section;
a camera (136) mounted on the distal face of the articulating section;
a data transmission wire (144) extending through the shaft between a distal end connected to the camera and a proximal end which remains external to the body for attachment to an external image processing device; and
a light source (138) at the distal face of the articulating section aimed to illuminate an area within a field of view of the camera
wherein the articulating section (116) defines an introduction lumen (132) and a withdrawal lumen (134) extending therethrough, the introduction lumen (132) being concentric with the withdrawal lumen (134).

2. The device of claim 1, wherein the articulating section is movable in any direction along at least one plane.

3. The device of claim 1, wherein movement of the articulating section is controlled by an actuator on a handle (102) mounted to a proximal end of the shaft.

4. The device of claim 3, wherein the actuator is formed as a control wheel (146) operably connected to a control wire extending from the control wheel to a distal end of the articulating section.

5. The device of claim 2, wherein the articulating section is formed of a plurality of substantially cylindrical elements (126) linked together so as to provide a substantially cylindrical articulating section.

6. The device of claim 5, wherein each of the cylindrical elements is movable out of longitudinal alignment with an adjacent one of the cylindrical elements.

7. The device of claim 5, wherein the articulating section is deflectable by approximately 90° from a longitudinal axis of the elongated shaft.

8. The device of claim 1, further comprising a seal (118) configured to prevent leakage of fluids
from a target body cavity, the seal being configured to permit temporary attachment of the articulating section to the shaft.

9. The device of claim 8, wherein the seal includes a plurality of flexible disks extending radially outward from an outer surface of the shaft.

10. The device of claim 1, further comprising a second shaft lumen (134) extending through the shaft and wherein the articulating section further comprises a second articulating section lumen extending between a proximal end open to a distal end of the second shaft lumen and a distal opening at a distal end of the articulating section.

11. The device of claim 1, wherein the shaft is sized and shaped for insertion through a cervix into a uterus for hydrothermal ablation.

12. The device of claim 1, wherein the camera is a Complimentary Metal-Oxide Semiconductor ("CMOS").

13. The device of claim 12, wherein the camera is no greater than 1 mm. x 1 mm. in size.

14. The device of claim 1, wherein an outer diameter of the articulating section is no greater than 6 mm.

## Patentansprüche

1. Vorrichtung (100), welche aufweist:
eine längliche Welle, die konfiguriert ist zur Einführung durch ein Körperlumen bis zu einer gewünschten Position innerhalb eines Zielkörperorgans, wobei die Welle ein erstes Wellenlumen (106), das sich durch diese erstreckt, definiert;
einen Gliederabschnitt (116), der sich an einem distalen Ende der Welle befindet, wobei der Gliederabschnitt unter der Steuerung durch einen Benutzer aus einer Längsausrichtung mit der Welle heraus bewegbar ist und der Gliederabschnitt ein erstes Gliederabschnittslumen (132) aufweist, das sich durch diesen von einem proximalen Ende, das zu dem ersten Wellenlumen hin offen ist, zu einer distalen Öffnung in einer distalen Fläche des Gliederabschnitts erstreckt;
eine Kamera (136), die an der distalen Fläche des Gliederabschnitts befestigt ist;
einen Datenübertragungsdraht (144), der sich durch die Welle zwischen einem distalen Ende, das mit der Kamera verbunden ist, und einem proximalen Ende, das außerhalb des Körpers verbleibt, erstreckt für die Anbringung an einer externen Bildverarbeitungsvorrichtung;
und
eine Lichtquelle (138) an der distalen Fläche des Gliederabschnitts, die zur Beleuchtung eines Raums innerhalb eines Sichtfelds der Kamera vorgesehen ist, wobei der Gliederabschnitt (116) ein Einführungslumen (132) und ein Rückzugslumen (134), die sich durch diesen erstrecken, definiert, und das Einführungslumen (132) konzentrisch mit dem Rückzugslumen (134) ist.

2. Vorrichtung nach Anspruch 1, bei der der Gliederabschnitt in jeder Richtung entlang zumindest einer Ebene bewegbar ist.

3. Vorrichtung nach Anspruch 1, bei der die Bewegung des Gliederabschnitts durch einen Aktuator an einem Handgriff (102), der an einem proximalen Ende der Welle befestigt ist, gesteuert wird.

4. Vorrichtung nach Anspruch 3, bei der der Aktuator als ein Steuerrad (146) gebildet ist, das betätigbar mit einem Steuerdraht verbunden ist, der sich von dem Steuerrad zu einem distalen Ende des Gliederabschnitts erstreckt.

5. Vorrichtung nach Anspruch 2, bei der der Gliederabschnitt aus mehreren im Wesentlichen zylindrischen Elementen (126) gebildet ist, die miteinander verbunden sind, um einen im Wesentlichen zylindrischen Gliederabschnitt zu erhalten.

6. Vorrichtung nach Anspruch 5, bei der jedes der zylindrischen Elemente aus einer Längsausrichtung mit einem benachbarten der zylindrischen Elemente heraus bewegbar ist.

7. Vorrichtung nach Anspruch 5, bei der der Gliederabschnitt um angenähert 90° gegenüber einer Längsachse der länglichen Welle auslenkbar ist.

8. Vorrichtung nach Anspruch 1, weiterhin aufweisend eine Abdichtung (118), die konfiguriert ist, das Entweichen von Fluiden aus einem Zielkörper-Hohlraum zu verhindern, wobei die Abdichtung konfiguriert ist, eine vorübergehende Anbringung des Gliederabschnitts an der Welle zuzulassen.

9. Vorrichtung nach Anspruch 8, bei der die Abdichtung mehrere flexible Scheiben enthält, die sich von einer äußeren Oberfläche der Welle aus radial auswärts erstrecken.

10. Vorrichtung nach Anspruch 1, weiterhin aufweisend ein zweites Wellenlumen (134), das sich durch die Welle erstreckt, wobei der Gliederabschnitt weiterhin ein zweites Gliederabschnittslumen aufweist, das sich zwischen einem proximalen Ende, das zu einem distalen Ende des zweiten Wellenlumens hin geöffnet ist, und einer distalen Öffnung an einem distalen Ende des Gliederabschnitts erstreckt.

11. Vorrichtung nach Anspruch 1, bei der die Welle für die Einführung durch einen Hals in eine Gebärmutter für hydrothermische Ablation bemessen und geformt ist.

12. Vorrichtung nach Anspruch 1, bei der die Kamera ein komplementärer Metalloxid-Halbleiter ("CMOS") ist.

13. Vorrichtung nach Anspruch 12, bei der die Kamera eine Größe von nicht mehr als 1 mm x 1 mm hat.

14. Vorrichtung nach Anspruch 1, bei der ein äußerer Durchmesser des Gliederabschnitts nicht größer als 6 mm ist.

## Revendications

1. Dispositif (100), comprenant :
un arbre allongé qui est configuré de manière à être inséré au travers d'une lumière de corps jusqu'à une position souhaitée à l'intérieur d'un organe de corps cible, l'arbre définissant une première lumière d'arbre (106) qui s'étend au travers ;
une section d'articulation (116) qui est située au niveau d'une extrémité distale de l'arbre, la section d'articulation pouvant être déplacée hors alignement longitudinal avec l'arbre sous la commande d'un utilisateur, la section d'articulation comprenant une première lumière de section d'articulation (132) qui s'étend au travers depuis une extrémité proximale qui débouche sur la première lumière d'arbre jusqu'à une ouverture distale qui est ménagée dans une face distale de la section d'articulation ;
une caméra (136) qui est montée sur la face distale de la section d'articulation ;
un fil de transmission de données (144) qui s'étend au travers de l'arbre entre une extrémité distale qui est connectée à la caméra et une extrémité proximale qui reste externe par rapport au corps pour une fixation sur un dispositif de traitement d'image externe ; et
une source de lumière (138) au niveau de la face distale de la section d'articulation qui est destinée à éclairer une zone à l'intérieur d'un champ visuel de la caméra ; dans lequel :
la section d'articulation (116) définit une lumière d'introduction (132) et une lumière de retrait (134) s'étendant au travers, la lumière d'introduction (132) étant concentrique à la lumière de retrait (134).

2. Dispositif selon la revendication 1, dans lequel la section d'articulation est mobile dans n'importe quelle direction suivant au moins un plan.

3. Dispositif selon la revendication 1, dans lequel le déplacement de la section d'articulation est commandé par un actionneur sur un manche/une poignée (102) qui est monté(e) sur une extrémité proximale de l'arbre.

4. Dispositif selon la revendication 3, dans lequel l'actionneur est formé en tant que roue de commande (146) qui est connectée de manière opérationnelle à un fil de commande qui s'étend depuis la roue de commande jusqu'à une extrémité distale de la section d'articulation.

5. Dispositif selon la revendication 2, dans lequel la section d'articulation est formée par une pluralité d'éléments sensiblement cylindriques (126) qui sont liés ensemble de manière à constituer une section d'articulation sensiblement cylindrique.

6. Dispositif selon la revendication 5, dans lequel chacun des éléments cylindriques peut être déplacé hors alignement longitudinal avec l'un adjacent des éléments cylindriques.

7. Dispositif selon la revendication 5, dans lequel la section d'articulation peut être déviée d'approximativement 90° par rapport à un axe longitudinal de l'arbre allongé.

8. Dispositif selon la revendication 1, comprenant en outre une étanchéité (118) qui est configurée de manière à empêcher une fuite de fluides depuis une cavité de corps cible, l'étanchéité étant configurée de manière à permettre une fixation temporaire de la section d'articulation sur l'arbre.

9. Dispositif selon la revendication 8, dans lequel l'étanchéité inclut une pluralité de disques flexibles qui s'étendent radialement vers l'extérieur depuis une surface externe de l'arbre.

10. Dispositif selon la revendication 1, comprenant en outre une seconde lumière d'arbre (134) qui s'étend au travers de l'arbre et dans lequel la section d'articulation comprend en outre une seconde lumière de section d'articulation qui s'étend entre une extrémité proximale qui débouche sur une extrémité distale de la seconde lumière d'arbre et une ouverture distale qui est ménagée au niveau d'une extrémité distale de la section d'articulation.

11. Dispositif selon la revendication 1, dans lequel l'arbre est dimensionné et conformé pour son insertion au travers d'un col de l'utérus à l'intérieur d'un utérus pour une ablation hydrothermique.

12. Dispositif selon la revendication 1, dans lequel la caméra est un métal oxyde semiconducteur complémentaire (« CMOS »).

13. Dispositif selon la revendication 12, dans lequel la taille de la caméra n'est pas plus grande que 1 mm x 1 mm.

14. Dispositif selon la revendication 1, dans lequel un diamètre externe de la section d'articulation n'est pas plus grand que 6 mm.
